# EUROPEAN PATENT SPECIFICATION

(11) **EP 1 716 881 B1**
(45) Date of publication and mention of the grant of the patent: **28.05.2008**
(21) Application number: 05026026.4
(22) Date of filing: 29.11.2005
(51) Int. Cl.: A61M 5/50, A61M 5/32, A61M 5/34

(54) **Safety syringe**
Sicherheitsspritze
Seringue de sécurité

(43) Date of publication of application: 02.11.2006
(73) Proprietor: Xiang Guang International Co., Ltd., Taichung City (TW)
(72) Inventor: Lai, Shui-Sheng, Taichung City (TW)
(74) Representative: Viering, Jentschura & Partner

(56) References cited:
- EP-A- 1 287 842
- EP-A- 1 514 566
- US-A- 6 066 115

## Description

### FIELD OF THE INVENTION

The present invention relates to syringes, and in particular to a safety syringe, wherein when a push rod is used to pull the syringe needle backwards, it will elastically reduce into the syringe needle of the syringe cylinder. Thereby the syringe needle is shifted and thus the syringe needle cannot be reused.

### BACKGROUND OF THE INVENTION

Syringes are necessary in current medical operations. Currently, the syringe needle is disposable for avoiding the syringe needle to be reused. However it is often that the used syringe needle cannot be well processed so as to hurt the medical persons.

To improve the defect, after injection, the syringe needle is pulled back to the syringe cylinder. In this improvement, which is disclosed in US-A-6,066,115, a seat in the syringe cylinder is screwed into a sleeve. A lower edge of the sleeve is formed with a tapered hole. A periphery of the hole is formed with a buckle. One side of the sleeve is downwards extended with a protrusion. When a tapered post at a front end of a liquid driving plug is pushed into the hole and resists against the buckle, the protrusion resists against an elastic plug. Then the liquid driving plug is returned to drive the syringe needle to reduce into the syringe cylinder. The elastic plug will provide an elastic force to the protrusion. Thereby the syringe needle shifts aside. The syringe needle at the front end of the syringe cylinder resists against the syringe cylinder. Thereby it cannot be pushed forwards. The syringe needle deserted will not hurt persons. Document US-A-6,066,115 discloses a safety syringe comprising: a syringe cylinder being a hollow cylinder; one end of the syringe cylinder being formed with an reduced section and another end thereof being an enlarged flange; an interior of the syringe cylinder being formed as a receiving chamber; an interior of the reduced section being formed with a receiving hole communicated to the receiving chamber; a push rod movably inserted in the receiving chamber of the syringe cylinder and movable linearly in the receiving chamber; an elastic plug being installed at one end of the push rod; the elastic plug being slidable against an inner wall of the receiving chamber of the syringe cylinder; one side of the elastic plug being installed with a tenon and another end thereof being formed with an annular ring;a needle set having a syringe needle, a seat and a sleeve; the syringe needle being inserted into and fixed to the seat; an outer expanded round buckling ring being extended from the seat; a lower side of the buckling ring of the seat being formed with an outer thread for locking the sleeve; the sleeve being received in the receiving hole; to tightly seal the sleeve in the receiving hole; the slave including a round combining section

However the prior art design has some defects necessary to be improved. A seat at a lower edge of the sleeve rests against an inner wall of the syringe cylinder by an O ring. When the syringe needle is pulled backwards, the protrusion cannot achieve to a predetermined distance. This is because the lower edge of the sleeve is confined by the O ring resisting against the inner wall of the syringe cylinder. Therefore no space is provided to shift the syringe needle.

Moreover, sleeve has an outward extended seat to engage into an interior of the syringe cylinder. Correspondingly, the seat protrudes from a receiving chamber of the syringe cylinder so that the syringe cylinder cannot store sufficient drug liquid. Although the syringe cylinder can be prolonged to increase the space for receiving the drug liquid, but this will increase cost in molding.

Further, if the length of the syringe cylinder is not prolonged, the drug liquid is not precisely controlled and the effect of the injection cannot be well controlled.

### SUMMARY OF THE INVENTION

Accordingly, the primary object of the present invention is to provide a safety syringe, wherein a maximum radius of the tapered section is smaller than the radius of the receiving chamber; a lower side of the tapered section is retained with a length from an inner wall of the syringe cylinder. Thereby the shift angle is achieved to an effective one. The syringe needle resists against the stop edge of the syringe cylinder so that the syringe needle cannot be exposed out. Thereby the injury from the pierce of the syringe needle is avoided.

Furthermore, when the tapered section at a lower edge of the sleeve is sleeved to a trumpet opening, the tapered section is flushed to the connection of the receiving chamber and the hole. The sleeve does not occupy the space of the receiving chamber of the syringe cylinder. Only the protrusion is inserted into the receiving chamber. Thereby the space of the receiving chamber for storing drug liquid is enlarged. This is beneficial for syringe filling drug liquid of only several cubic centimeters.

Moreover, in the present invention, the O ring positioned to the upper side of the tapered section, so that the sleeve resists against the wall of the receiving hole so as to prevent from permeation.

To achieve above objects, the present invention provides a safety syringe. The safety syringe comprises an syringe cylinder; an interior of the syringe cylinder being formed as a receiving chamber; an interior of the reduced section being formed with a receiving hole communicated to the receiving chamber; a push rod movably inserted in the receiving chamber of the syringe cylinder and movably linearly in the receiving chamber; an elastic plug being installed at one end of the push rod; a needle set having a syringe needle, a seat and a sleeve; the syringe needle being inserted into and fixed to the seat; an outer expanded round buckling ring being extended from the seat; a lower side of the buckling ring of the seat being formed with an outer thread for locking the sleeve; the sleeve being received in the receiving hole; a periphery at a bottom of the tapered section being extended downwards with a protrusion; when the tapered section at a lower edge of the sleeve is sleeved to the trumpet opening, the tapered section is flushed to the connection of the receiving chamber and the receiving hole; only the protrusion protruding from the receiving chamber; wherein when the injection operation is complete, the protrusion resisted against the elastic plug.

The various objects and advantages of the present invention will be more readily understood from the following detailed description when read in conjunction with the appended drawing.

### BRIEF DESCRIPTION OF THE DRAWINGS

Fig. 1 is an exploded perspective view of the safety syringe of the present invention.
Fig. 2 is an exploded schematic view of the safety syringe of the present invention.
Fig. 2A is a schematic cross sectional view about the sleeve of the safety syringe of the present invention.
Fig. 3 shows the schematic cross sectional view about the application of the safety syringe of the present invention.
Fig. 4 is a schematic cross sectional view about the pull backward operation of the safety syringe of the present invention.
Fig. 5 is a schematic view showing the shift of the syringe needle of the safety syringe of the present invention.
Fig. 6 is schematic cross sectional view about the second embodiment of the safety syringe of the present invention.
Fig. 7 shows the schematic view about the sleeve of the second embodiment of the safety syringe of the present invention.

### DETAILED DESCRIPTION OF THE INVENTION

In order that those skilled in the art can further understand the present invention, a description will be described in the following in details. However, these descriptions and the appended drawings are only used to cause those skilled in the art to understand the objects, features, and characteristics of the present invention, but not to be used to confine the scope of the present invention defined in the appended claims.

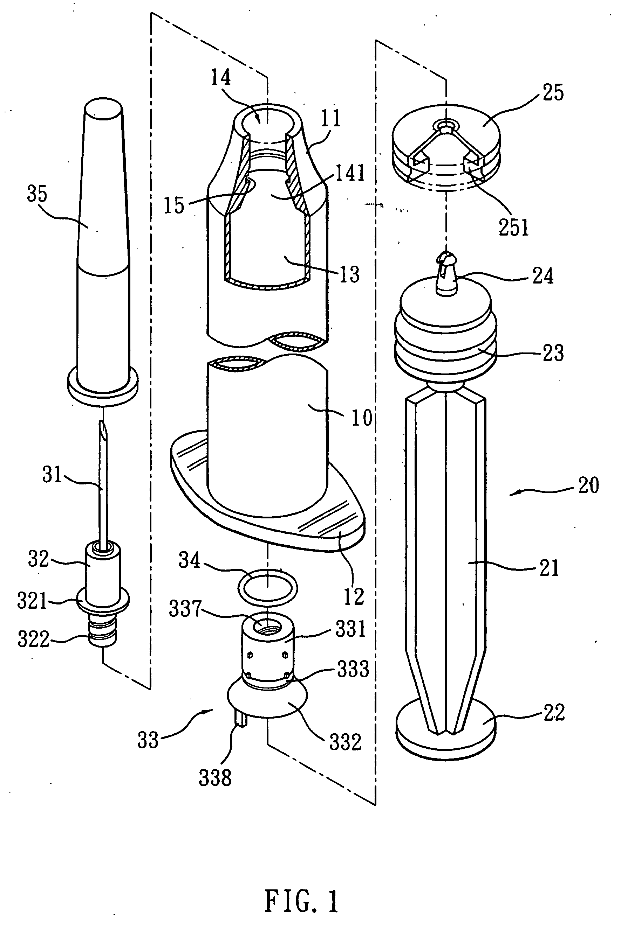

Referring to Fig. 1, the safety syringe of the present invention is illustrated. The present invention has the following elements.

An syringe cylinder 10 is a hollow cylinder. One end of the syringe cylinder 10 is formed with a reduced section 11 and another end thereof is an enlarged flange 12. An interior of the syringe cylinder 10 is formed as a receiving chamber 13. An interior of the reduced section 11 is formed with a receiving hole 14 which has a reduced hole diameter and is communicated to the receiving chamber 13. A diameter of the receiving chamber 13 is slightly larger than that of the receiving hole 14. The connection of the receiving chamber 13 and the receiving hole 14 is formed with a stop edge. The receiving hole 14 is formed with a trumpet opening 141 which has a smaller end at the outer side and has a larger end at the end connected to the receiving chamber 13. A periphery wall of the trumpet opening 141 is formed with a buckling groove 15.

A push rod 20 is movably inserted in the receiving chamber 13 of the syringe cylinder 10 and is movable linearly in the receiving chamber 13. A body 21 of the push rod 20 has an enlarged distal end which is formed as an annular ring. A front end thereof is formed with a combining portion 23 which is formed by a plurality of spaced disk. A front end of the combining portion 23 extends with a tenon 24. An elastic plug 25 encloses the tenon 24 and then is installed to the combining portion 23.

A needle set having a syringe needle 31, a seat 32 and a sleeve 33. The syringe needle 31 is inserted and fixed to the seat 32. An outer expanded round buckling ring 321 is extended from the seat 32. A cover 35 resists against the seat 32 so as to cover upon the syringe needle 31. A lower side of the buckling ring 321 of the seat 32 is formed with an outer thread 322 for locking the sleeve 33.

The sleeve 33 is received in the receiving hole 14. To tightly seal the sleeve 33 in the receiving hole 14, the sleeve 33 is formed as a trumpet to match the shape of the receiving hole 14. The sleeve 33 includes a round combining section 331 and a tapered section 332. The connection of the combining section 331 and the tapered section 332 is formed with an annular recess 333. A rubber O ring 34 is sleeved into the annular recess 333. The O ring 34 is also located at the buckling groove 15 of the wall of the trumpet opening 141 so that the sleeve 33 is tightly engaged to the receiving hole 14 and is not separated from the injection direction. Besides the maximum diameter of the tapered section 332 is smaller that the diameter of the receiving chamber 13.

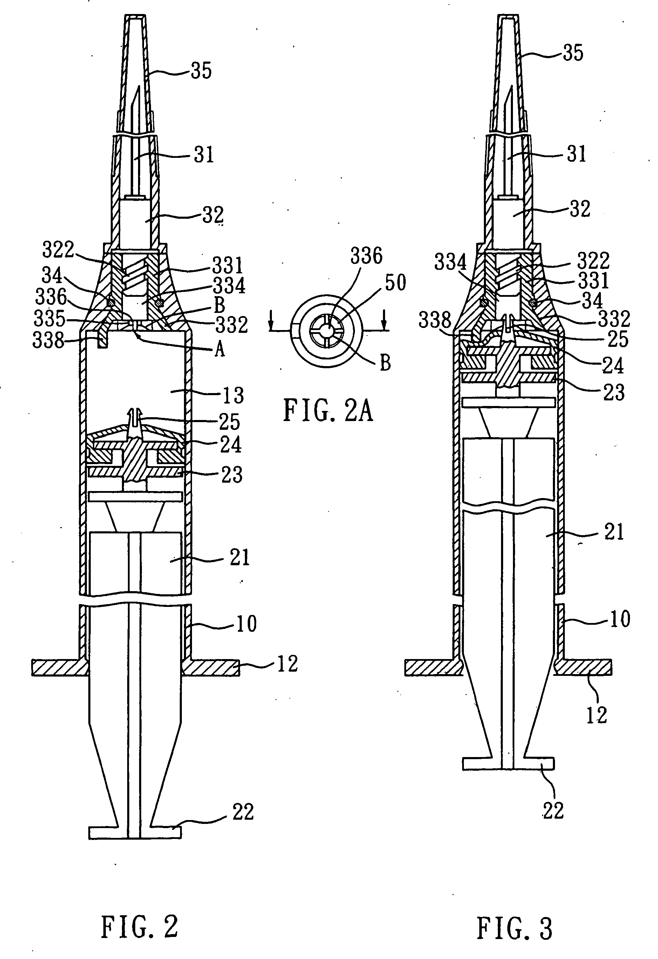

With reference to Figs. 2 and 3, the sleeve 33 is formed with an axial penetrating receiving portion 334. The receiving portion 334 is communicated to the tapered hole A at a lower side of the tapered section 332. A periphery of the tapered hole A is formed with a plurality of buckles B which are equally spaced. Each buckle B is a buckling portion 335 protruded from a wall of the tapered hole A. A middle section of the buckle B is formed with a through hole 336, as shown in Fig. 2A. In use, the tenon is inserted into the tapered hole A so that the tenon passes through the through hole 112 to resist against the buckling portion. The elastic tenon 34 passes through the through hole 336 to be received in the receiving portion 334. Then the tenon 34 cannot be drawn out since the tenon 24 is returned to the original position.

Furthermore, an inner thread 337 at a wall of the receiving portion 334 is screwed by the outer thread 322 of the seat 32 to a periphery at a bottom of the tapered section 332. A periphery at a bottom of the tapered section 332 is extended downwards with a protrusion 338. Normally, the protrusion 338 protrudes into the receiving chamber 13. When the push rod is completely pushed into the syringe cylinder, the elastic plug 25 at the front end of the push rod elastically resists against the protrusion 338. However the structure of the safety syringe according to the present invention is illustrated. The effect of the present invention will be described in the following.

The maximum radius of the tapered section is smaller than the radius of the receiving chamber of the syringe cylinder 10. Therefore when pulling the syringe needle 31 downwards, a lower edge of the tapered section is retained with a predetermined distance to an inner wall of the syringe cylinder 10 so as to have a preferred effect of shift of the syringe needle.

Besides when the tapered section 332 at a lower edge of the sleeve 33 is sleeved to the trumpet opening 141, the tapered section 332 is flushed to the connection of the receiving chamber 13 and the hole. Only the protrusion 338 protrudes from the receiving chamber 13. Other than the protrusion 338, the sleeve 33 is received in the receiving hole 14. Thereby the space of the receiving chamber 13 for storing drug liquid is enlarged. This is a beneficial for syringe filling drug liquid of only several cubic centimeters.

With reference to Fig. 3, when the push rod 20 is pushed to a predetermined position, the tenon 24 will pass through the through hole 336 to be received in the receiving portion 334 and resist against an upper end of the buckling portion 335. The elastic plug 25 at the front end of the push rod resists against the protrusion 338 protruded from the receiving chamber 13 so as to deform slightly.

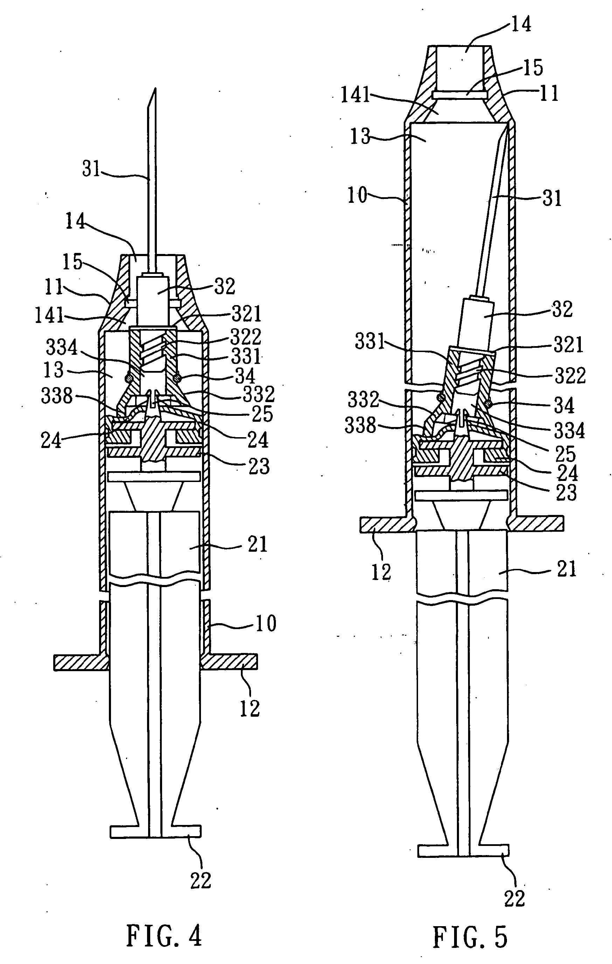

Referring to Figs. 4 and 5, when the push rod 20 is pushed back, the syringe needle will be pulled to separated from the receiving hole 14. If the syringe needle 31 is pulled back continuously until the syringe needle reduces to the receiving chamber of the syringe cylinder as shown in Fig. 5. The deformed elastic plug 25 will push the protrusion 338 so that the needle reduced into the receiving chamber 13 will shift aside. A lower side of the tapered section is retained with a length from an inner wall of the syringe cylinder 10. Thereby the shift angle is achieved to an effective one. The syringe needle 31 resists against the stop edge of the syringe cylinder 10 so that the syringe needle 31 cannot be exposed out. Thereby the hurt from the pierce of the syringe needle 31 is avoided.

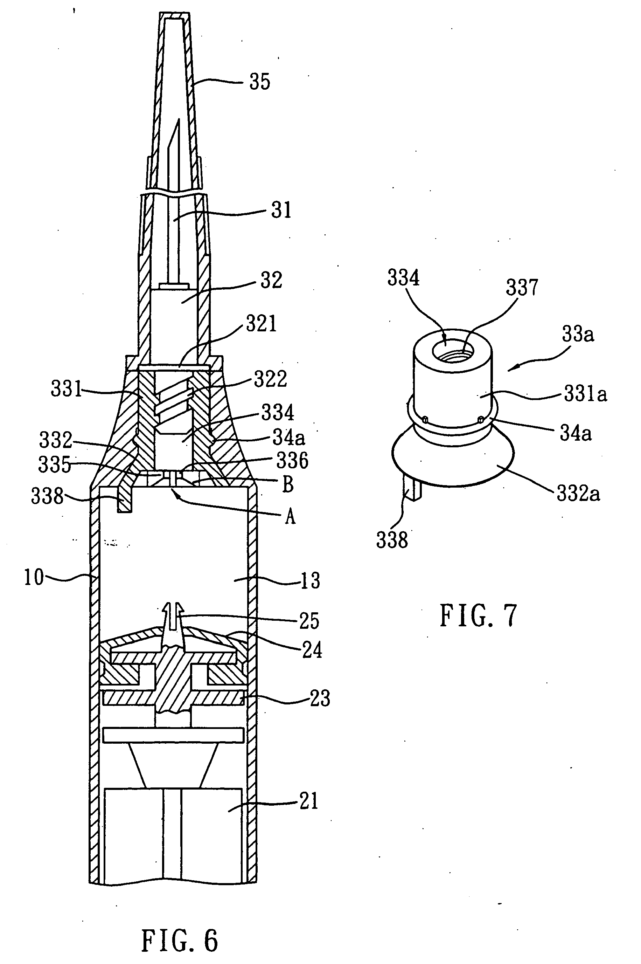

With reference to Figs. 6 and 7, the second embodiment of the safety syringe of the present invention is illustrated. Those identical from the above embodiment will not described herein. Only those different are described herein.

In Fig. 7, at a connection of the combining section 331 a of the sleeve 33a and the tapered section 332a, an annular ring 34 extends from the connection so that the sleeve 33a will be sleeved into the receiving hole 14. Thereby by the annular ring to buckle with the buckling groove 15 of the receiving hole 14, see Fig. 6, the combination of the syringe needle 31 and the syringe cylinder 10 is enhanced.

Besides, it should be noted that in the present invention, the trumpet hole and the tapered section are engaged. It is only necessary to engage the sleeve 33 into the receiving hole 14 so that the connection of the combining section 331 with the tapered section 332 and the trumpet opening 141 are matched so that the assembly work can be performed rapidly and accurately.

The present invention is thus described, it will be obvious that the same may be varied in many ways. Such variations are not to be regarded as a departure from the scope of the present invention, and all such modifications as would be obvious to one skilled in the art are intended to be included within the scope of the following claims.

## Claims

1. A safety syringe comprising:
a syringe cylinder (10) being a hollow cylinder; one end of the syringe cylinder (10) being formed with an reduced section (11) and another end thereof being an enlarged flange (12); an interior of the syringe cylinder (10) being formed as a receiving chamber (13); an interior of the reduced section (11) being formed with a receiving hole (14) communicated to the receiving chamber (13);
a push rod (20) movably inserted in the receiving chamber (13) of the syringe cylinder (10) and movable linearly in the receiving chamber (13); an elastic plug (25) being installed at one end of the push rod (20); the elastic plug (25) being slidable against an inner wall of the receiving chamber (13) of the syringe cylinder (10); one side of the elastic plug (25) being installed with a tenon (24) and another end thereof being formed with an annular ring;
a needle set having a syringe needle (31), a seat (32) and a sleeve (33, 33a); the syringe needle (31) being inserted into and fixed to the seat (32); an outer expanded round buckling ring (321) being extended from the seat (32); a lower side of the buckling ring (321) of the seat (32) being formed with an outer thread (322) for locking the sleeve (33);
the sleeve (33) being received in the receiving hole (14); to tightly seal the sleeve (33, 33a) in the receiving hole (14); the sleeve (33) including a round combining section (331, 331a)
**characterized in**
the receiving hole (14) being formed with a trumpet opening (141) which has a smaller end at the outer side and has a larger end at the end connected to the receiving chamber (13); the sleeve (33) being formed with a trumpet shape to match the shape of the receiving hole (14); the sleeve (33) including a tapered section (332, 332a); a maximum radius of the tapered section (332, 332a) being smaller than the radius of the receiving chamber (13); a periphery at a bottom of the tapered section (332, 332a) being extended downwards with a protrusion(338); when the tapered section (332, 332a) at a lower edge of the sleeve (33, 33a) is sleeved to the trumpet opening (141), the tapered section (332, 332a) is flushed to the connection of the receiving chamber (13) and the receiving hole (14); only the protrusion (338) protruding from the receiving chamber (13); wherein when the injection operation is complete, the protrusion (338) resists against the elastic plug (25).

2. The safety syringe as claimed in claim 1, wherein the tapered section (332, 332a) is formed with a buckle portion (335) near a lower wall thereof; the buckle portion (335) is configured to match to the tenon (24).

3. The safety syringe as claimed in claim 1, wherein a periphery wall of the trumpet opening is formed with a buckling groove (15); the sleeve (33) is formed with an annular recess (333); by an O ring (34) to be engaged to the buckling groove (15) and the annular recess (333), the sleeve (33) is positioned in the receiving hole (14).

4. The safety syringe as claimed in claim 1, wherein an inner wall of the receiving hole (14) is formed with a buckling groove (15); and the sleeve (33a) is integrally installed with an annular ring (34a); by the annular ring (34a) to be received into the buckling groove (15), the sleeve (34) is positioned in the receiving hole (14).

5. The safety syringe as claimed in claim 1, wherein the sleeve (34, 34a) is made of rubber.

## Patentansprüche

1. Sicherheitsspritze, aufweisend:
einen Spritzenzylinder (10), der ein Hohlzylinder ist, wobei an einem Ende des Spritzenzylinders (10) ein reduzierter Abschnitt (11) geformt ist und ein anderes Ende davon ein vergrößerter Flansch (12) ist, wobei ein Innenraum des Spritzenzylinders (10) als eine Aufnehmkammer (13) geformt ist, wobei ein Innenraum des reduzierten Abschnitts (11) mit einem Aufnehmloch (14) geformt ist, das in Verbindung mit der Aufnehmkammer (13) ist,
eine Schubstange (20), die bewegbar in die Aufnehmkammer (13) des Spritzenzylinders (10) eingeführt ist und gradlinig in der Aufnehmkammer (13) bewegbar ist, einen elastischen Stöpsel (25), der an einem Ende der Schubstange (20) installiert ist, wobei der elastische Stöpsel (25) entlang einer inneren Wand der Aufnehmkammer (13) des Spritzenzylinders (10) verschiebbar ist, wobei eine Seite des elastischen Stöpsels (25) mit einem Zapfen (24) versehen ist und ein anderes Ende davon mit einem kranzförmigen Ring geformt ist,
ein Nadelset, aufweisend: eine Spritzennadel (31), einen Sitz (32) und einen Ärmel (33, 33a), wobei die Spritzennadel in den Sitz (32) eingeführt ist und daran befestigt ist, einen nach außen ausgedehnten runden Abdeckring (321), wobei eine untere Seite des Abdeckrings (321) des Sitzes (32) mit einem Außengewinde (322) geformt ist zum Befestigen des Ärmels (33), wobei
der Ärmel (33) in dem Empfangsloch (14) aufgenommen wird, um den Ärmel (33, 33a) in dem Empfangsloch (14) fest abzudichten, wobei der Ärmel (33) einen runden Verbindungsabschnitt (331, 331a) aufweist,
**gekennzeichnet dadurch, dass**
das Empfangsloch (14) mit einer Trichteröffnung (141) geformt ist, die ein kleineres Ende an der äußeren Seite aufweist und ein größeres Ende an dem Ende, das mit der Aufnehmkammer (13) verbunden ist, wobei der Ärmel (33) in einer Trichterform geformt ist, um sich der Form des Aufnehmlochs (14) anzupassen, wobei der Ärmel (33) einen konischförmigen Abschnitt (332, 332a) aufweist, wobei ein maximaler Radius des konischförmigen Abschnitts (332, 322a) kleiner ist als der Radius der Aufnehmkammer (13), wobei ein Umfang an einer unteren Seite des konischförmigen Abschnitts (322, 322a) sich nach unten mit einem Vorsprung (338) erstreckt, wobei, wenn der konischförmige Abschnitt (332, 332a) an einem unteren Rand des Ärmels (33, 33a) mit der Trichteröffnung (141) verbunden ist, der konischförmige Abschnitt (332, 332a) mit der Aufnehmkammer (13) und mit dem Aufnehmloch (14) bündig ist, wobei nur der Vorsprung (338) von der Aufnehmkammer (13) hervorsteht, wobei, wenn der Injektionsvorgang fertig ist, der Vorsprung (338) gegen den elastischen Stöpsel (25) steht.

2. Sicherheitsspritze nach Anspruch 1, wobei der reduzierte Abschnitt (332, 322a) mit einem Klemmteil (335) in der Nähe einer unteren Wand davon geformt ist, wobei das Klemmteil (335) derart konfiguriert ist, auf den Zapfen zu passen.

3. Sicherheitsspritze nach Anspruch 1, wobei eine Umfangswand der Trichteröffnung mit einer Klemmnut (15) geformt ist, wobei der Ärmel (33) mit einer kranzförmigen Aussparung (333) geformt ist, wobei der Ärmel (33) mittels eines O-Rings (34), der in Engriff mit der Klemmnut (15) und der kranzförmigen Aussparung (333) ist, in dem Aufnehmloch (14) positioniert ist.

4. Sicherheitsspritze nach Anspruch 1, wobei die innere Wand des Aufnehmlochs (14) mit einer Klemmnut (15) geformt ist, und der Ärmel (33a) einheitlich mit einem kranzförmigen Ring (34a) installiert ist, wobei der kranzförmige Ring (34a) in der Klemmnut (15) aufzunehmen ist, der Ärmel (34) in dem Aufnehmloch (14) positioniert wird.

5. Sicherheitsspritze nach Anspruch 1, wobei der Ärmel (34, 34a) aus Gummi ist.

## Revendications

1. Seringue de sécurité comprenant :
un cylindre de seringue (10) qui est un cylindre creux ; une extrémité du cylindre de seringue (10) étant formée avec une section réduite (11) et une autre extrémité de celui-ci étant une collerette élargie (12) ; un intérieur du cylindre de seringue (10) étant formé en tant que chambre de réception (13) ; un intérieur de la section réduite (11) étant formé avec un trou de réception (14) communiquant vers la chambre de réception (13) ;
une tige-poussoir (20) insérée de manière mobile dans la chambre de réception (13) du cylindre de seringue (10) et pouvant se déplacer de façon linéaire dans la chambre de réception (13) ; un bouchon élastique (25) étant installé à une extrémité de la tige-poussoir (20) ; le bouchon élastique (25) pouvant coulisser contre une paroi interne de la chambre de réception (13) du cylindre de seringue (10) ; un côté du bouchon élastique (25) étant muni d'un tenon (24) et l'autre extrémité de celui-ci étant formée avec une bague annulaire ;
un ensemble d'aiguille présentant une aiguille de seringue (31), un siège (32) et un manchon (33, 33a) ; l'aiguille de seringue (31) étant insérée dans et fixée au siège (32) ; une bague d'attache arrondie expansée externe (321) s'étendant depuis le siège (32) ; un côté inférieur de la bague d'attache (321) du siège (32) étant formé avec un filetage externe (322) pour bloquer le manchon (33) ;
le manchon (33) étant reçu dans le trou de réception (14) ; pour sceller de manière étanche le manchon (33, 33a) dans le trou de réception (14) ; le manchon (33) comprenant une section de combinaison arrondie (331, 311 a)
**caractérisée en ce que**
le trou de réception (14) est formé avec une ouverture en trompette (141) qui présente une extrémité inférieure du côté externe et une extrémité plus importante à l'extrémité raccordée à la chambre de réception (13) ; le manchon (33) étant conçu en forme de trompette pour correspondre à la forme du trou de réception (14) ; le manchon (33) comprenant une section conique (332, 322a) ; un rayon maximum de la section conique (332, 332a) étant inférieur au rayon de la chambre de réception (13) ; une périphérie au niveau d'un fond de la section conique (332, 332a) s'étendant vers le bas avec une saillie (338) ; lorsque la section conique (332, 332a) à un bord inférieur du manchon (33, 33a) est emmanchée dans l'ouverture en trompette (141), la section conique (332, 332a) est en affleurement avec le raccord de la chambre de réception (13) et du trou de réception (14) ; seule la saillie (338) faisant saillie depuis la chambre de réception (13) ; dans laquelle lorsque l'opération d'injection est réalisée, la saillie (338) résiste au bouchon élastique (25).

2. Seringue de sécurité selon la revendication 1, dans laquelle la section conique (332, 332a) est formée avec une partie d'attache (335) à proximité d'une paroi inférieure de celle-ci ; la partie d'attache (335) est configurée pour correspondre au tenon (24).

3. Seringue de sécurité selon la revendication 1, dans laquelle une paroi périphérique de l'ouverture de trompette est formée avec une rainure d'attache (15) ; le manchon (33) est formé avec un évidement annulaire (333) ; grâce à un joint torique (34) destiné à venir en prise avec la rainure d'attache (15) et l'évidement annulaire (333), le manchon (33) est positionné dans le trou de réception (14).

4. Seringue de sécurité selon la revendication 1, dans laquelle une paroi interne du trou de réception (14) est formée avec une rainure d'attache (15) ; et le manchon (33a) est installé de manière solidaire avec une bague annulaire (34a) ; grâce à la bague annulaire (34a) destinée à être reçue dans la rainure d'attache (15), le manchon (34) est positionné dans le trou de réception (14).

5. Seringue de sécurité selon la revendication 1, dans laquelle le manchon (34, 34a) est constitué de caoutchouc.
